(19) Europäisches Patentamt
European Patent Office
Office européen des brevets

(11) Veröffentlichungsnummer: **0 010 745**
**B1**

(12) # EUROPÄISCHE PATENTSCHRIFT

(45) Veröffentlichungstag der Patentschrift: **13.04.83**

(51) Int. Cl.³: **C 07 D 211/46,** A 61 K 31/445, A 23 K 1/16

(21) Anmeldenummer: **79104154.4**

(22) Anmeldetag: **26.10.79**

(54) **Derivate des 2-Hydroxymethyl-3,4,5-trihydroxy-piperidins, ihre Herstellung und sie enthaltende Arzneimittel.**

(30) Priorität: **06.11.78 DE 2848117**

(43) Veröffentlichungstag der Anmeldung: **14.05.80 Patentblatt 80/10**

(45) Bekanntmachung des Hinweises auf die Patenterteilung: **13.04.83 Patentblatt 83/15**

(84) Benannte Vertragsstaaten: **AT BE CH DE FR GB IT NL SE**

(56) Entgegenhaltungen:
**EP-A-0 000 947**
**FR-A-2 336 941**
**CHEMISCHE BERICHTE, Jahrgang 100, Nr. 8, 1967, Seiten 2467—2473 Weinheim, DE. H. PAULSEN et al.: »Darstellung von 5-Hydrazino-5-desoxy-D-xylose. Umwandlung in N-Amino-D-xylopiperidinose und ein dimeres Hexahydrotetrazin-Derivat«**

(73) Patentinhaber: **BAYER AG, Zentralbereich Patente, Marken und Lizenzen, D-5090 Leverkusen 1, Bayerwerk (DE)**

(72) Erfinder: **Böshagen, Horst, Dr., Wiesenstrasse 4, D-5657 Haan (DE)**
Erfinder: **Sitt, Rüdiger, Dr., Claudiusweg 9, D-5600 Wuppertal 1 (DE)**
Erfinder: **Truscheit, Ernst, Dr., Horather Strasse 160, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Derivate des 2-Hydroxymethyl-3,4,5-trihydroxy-piperidins, ihre Herstellung und sie enthaltende Arzneimittel

Die Erfindung betrifft in 6-Stellung substituierte Derivate des 2-Hydroxymethyl-3,4,5-trihydroxy-piperidins, ihre Herstellung sowie ihre Verwendung als Arzneimittel zur Beeinflussung des Kohlenhydrat- und Fettstoffwechsels und ihre Verwendung als Futterzusatzmittel in der Tierernährung.

Die erfindungsgemäßen Verbindungen entsprechen der Formel (I)

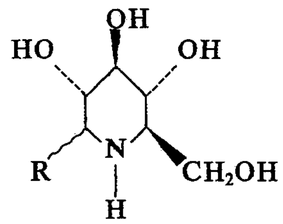

(I)

in der

R einen $C_1-C_{18}$-Alkyl-, $C_2-C_{18}$-Alkenyl- oder Alkinylrest darstellt.

Als Substituenten der übrigen Definitionen von R seien beispielhaft genannt $C_1-C_4$-Alkyl, $C_1-C_4$-Alkoxy, $C_1-C_4$-Alkylmercapto, Halogen, insbesondere Fluor, Chlor und Brom, Di-$C_1-C_{12}$-Alkylamino, Pyrrolidino, Piperidino, Morpholino, N'-$C_1-C_4$-alkylpiperazino, gegebenenfalls durch Halogen, insbesondere Fluor, Chlor und Brom, $C_1-C_4$-Alkyl, $C_1-C_4$-Alkoxy, $C_1-C_4$-Alkylmercapto,$C_1-C_4$-Alkylsulfonyl, Nitro, $C_1-C_4$-Alkoxycarbonyl, Di-$C_1-C_{12}$-Alkylaminocarbonyl, Di-$C_1-C_{12}$-Alkylaminosulfonyl, Pyridyl, Thienyl, Imidazolyl, Isoxazolyl, Thioazolyl, Glucopyranosyl und Ribofuranosyl substituiertes Phenyl oder Naphthyl.

Die Alkylreste R können darüber hinaus $C_3-C_{12}$-Cycloalkyl, -Cycloalkenyl, -Cycloalkadienyl, -Bicycloalkyl, -Bicycloalkenyl, -Bicycloalkadienyl, -Tricycloalkyl, -Tricycloalkenyl oder -Tricycloalkadienyl als Substituenten tragen.

In bevorzugten Verbindungen der Formel (I) bedeutet R $C_1-C_{18}$-Alkyl, gegebenenfalls durch 1 bis 5 Halogenatome substituiert, $C_3-C_{10}$-Alkenyl, $C_3-C_{10}$-Alkinyl, $C_3-C_7$-Cycloalkyl oder gegebenenfalls durch Halogen, $C_1-C_4$-Alkyl, $C_1-C_4$-Alkoxy oder Nitro substituiertes Phenyl.

Die Verbindungen der Formel (I) werden erhalten, indem man 2-Hydroxymethyl-3,4,5-trihydroxy-6-cyano-piperidin durch Erwärmen mit Hexaalkyl-disilazanen oder Trialkylsiliciumhalogeniden unter Zusatz eines Katalysators an den OH-Gruppen silyliert, wobei Alkyl vorzugsweise $C_1-C_4$-Alkyl und Halogen vorzugsweise Chlor ist, Hexamethyldisilazan ist bevorzugt, mit einem geeigneten metallorganischen Reagenz der Formel (II)

$$R-Y \qquad (II)$$

worin

R die obengenannte Bedeutung hat und
Y ein Metall oder Halogenmetall bedeutet,

umsetzt und die Silyl-Schutzgruppen abspaltet.

Man erhält ein trennbares Gemisch aus der $\alpha$- und $\beta$-Verbindung, wobei das $\alpha$-Derivat überwiegt. Die Wirksamkeiten von $\alpha$- und $\beta$-Form unterscheiden sich nur wenig, wobei die $\alpha$-Form im allgemeinen etwas wirksamer ist. Dabei wird als $\alpha$-Form diejenige Verbindung bezeichnet, die im Laufmittel Essigsäureäthylester/Methanol/Wasser/$NH_4OH$ (100/60/25/1) auf DC-Fertigplatten Merck, Kieselgel 60 F 254, den kleineren Rf-Wert besitzt.

Wie aus dem folgenden Formelschema hervorgeht, wird in einem chemisch eigenartigen Verfahren überraschenderweise die Cyangruppe durch den jeweils beabsichtigten Alkylrest substituiert, wobei die wirksame $\alpha$-Form in wesentlich höherer Ausbeute als die $\beta$-Form gewonnen wird. Eine Aromatisierung zum Pyridin-Derivat tritt nicht ein.

Als Katalysatoren für die Silylierung kommen Imidazol und 1,2,4-Triazol infrage. Die Trennung des $\alpha$-Derivates vom $\beta$-Derivat erfolgt mit Kationenaustauschern, z. B. Dowex 50 WX 4.

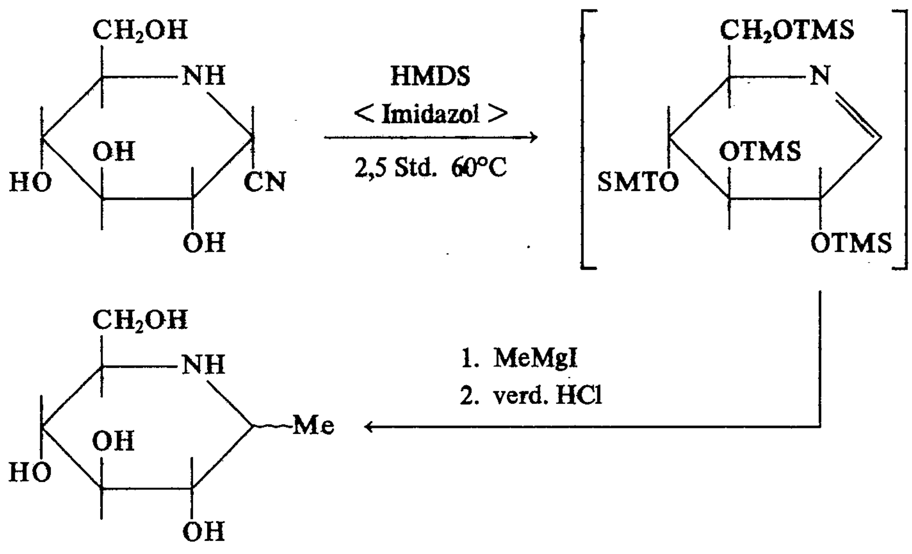


HMDS = Hexamethyldisilazan

TMS = Trimethylsilyl

Die Umsetzung mit Hexamethyldisilazan erfolgt unter Zusatz von Imidazol (~0,1 Mol-Äquivalent) bei einer Temperatur von 20–125°C, vorzugsweise 60°C, im Laufe von 2–18 Stunden, wobei ein Überschuß an Hexamethyldisilazan gleichzeitig als Lösungsmittel dient. Anschließend wird überschüssiges Hexamethyldisilazan im Vakuum abgezogen, der Rückstand in einem inerten Lösungsmittel, vorzugsweise Diethylether gelöst und mit einem metallorganischen Reagenz, gelöst in einem inerten Lösungsmittel, z. B. Ether, Petrolether, Ligroin, bei einer Temperatur von 20–60°C, vorzugsweise Raumtemperatur, umgesetzt. Anschließend werden die Silylgruppen durch 18-stündige Behandlung der Reaktionslösung mit verdünnter Salzsäure (vorzugsweise 1 N-HCl) bei Raumtemperatur entfernt. Die wäßrige Phase wird abgetrennt, mit Natronlauge neutralisiert und eingedampft. Das so erhaltene Rohprodukt wird mittels eines Kationenaustauschers von beigemengten Salzen befreit und anschließend in geeigneter Weise weiter gereinigt.

2-Hydroxymethyl-3,4,5-trihydroxy-6-cyanopiperidin wird aus 5-Amino-5-desoxy-D-glucose-1-sulfosäure (S. Inouye et. al., Tetrahedron 23, 2143 [1968]) und Cyanwasserstoffsäure analog der Herstellung von 2,6-Imino-2-hydroxymethyl-2,6-didesoxy-L-ido-hexonsäurenitril (H. Paulsen et. al., Chem. Ber. 100, 812 [1967]) dargestellt.

Die eingesetzten metallorganischen Verbindungen sind bekannt. Als Beispiele seien n-Nonylmagnesiumbromid, Undecyl-(2)-magnesiumbromid, 2-Methyl-butyl-(2)-magnesiumchlorid, Vinyl-magnesiumbromid, Propenyl-magnesiumbromid, Allyl-magnesiumbromid, Propargyl-magnesiumbromid, Myrtenyl-magnesiumchlorid oder deren lithiumorganische Äquivalente genannt.

Es wurde gefunden, daß die neuen Verbindungen der Formel I potente Inhibitoren für $\alpha$-Glucosidasen, insbesondere für Disaccharidasen sind. Daher sind die neuen Verbindungen wertvolle Mittel zur Beeinflussung einer Vielzahl von Stoffwechselvorgängen und bereichern somit den Arzneimittelschatz. Gegenüber dem aus der DE-OS 2 656 602 bekannten 2-Hydroxymethyl-3,4,5-trihydroxypiperidin weisen die neuen Verbindungen vorteilhafte therapeutische Eigenschaften auf.

Die erfindungsgemäßen Inhibitoren eignen sich als Therapeutica für folgende Indikationen:

Prädiabetes, Gastritis, Obstipation, Karies, Atheroskelerose und besonders Adipositas, Diabetes und Hyperlipoprotämie.

Zur Verbreiterung des Wirkungsspektrums kann es sich empfehlen, Inhibitoren für Glycosidhydrolasen, die sich gegenseitig in ihrer Wirkung ergänzen, zu kombinieren, sei es, daß es sich um Kombinationen der erfindungsgemäßen Inhibitoren untereinander oder um Kombinationen der erfindungsgemäßen Inhibitoren mit bereits bekannten handelt. So kann es beispielsweise zweckmäßig sein, erfindungsgemäße Saccharase-Inhibitoren mit bereits bekannten Amylase-Inhibitoren zu kombinieren.

Vorteilhaft sind in manchen Fällen auch Kombinationen der erfindungsgemäßen Inhibitoren mit bekannten oralen Antidiabetica ($\beta$-cytotrope Sulfonylharnstoffderivate und/oder blutzuckerwirksame Biguanide) sowie mit blutlipidsenkenden Wirkstoffen wie Clofibrat, Nicotinsäure, Cholestyramin und anderen.

Die Verbindungen können ohne Verdünnung, z. B. als Pulver oder in einer Gelatinehülle oder in Kombination mit einem Trägerstoff in einer pharmazeutischen Zusammensetzung appliziert werden.

Pharmazeutische Zubereitungen können eine größere oder kleinere Menge des Inhibitors enthalten, z. B. 0,1% bis 99,5%, in Kombination mit einem pharmazeutisch verträglichen nichttoxischen, inerten

Trägerstoff, wobei der Trägerstoff eine oder mehrere feste, halbfeste oder flüssige Verdünnungsmittel, Füllstoffe und/oder oder nichttoxische, inerte und pharmazeutisch-verträgliche Formulierungshilfsmittel enthalten kann. Solche pharmazeutischen Zubereitungen liegen vorzugsweise in Form von Dosierungseinheiten vor, d. h. physikalisch-diskreten, eine bestimmte Menge des Inhibitors enthaltenden Einheiten, die einem Bruchteil oder einem Vielfachen der Dosis entsprechen, die zur Herbeiführung der gewünschten Hemmwirkung erforderlich sind. Die Dosierungseinheiten können 1, 2, 3, 4 oder mehr Einzeldosen oder $^1/_2$, $^1/_3$ oder $^1/_4$ einer Einzeldosis enthalten. Eine Einzeldosis enthält vorzugsweise eine genügende Menge Wirkstoff, um bei einer Applikation gemäß eines vorher bestimmten Dosierungsschemas einer oder mehrerer Dosierungseinheiten die gewünschte Hemmwirkung zu erzielen, wobei eine ganze, eine halbe, oder ein Drittel oder ein Viertel der Tagesdosis gewöhnlich zu allen, Haupt- und Nebenmahlzeiten am Tage verabreicht wird. Andere therapeutische Mittel können auch eingenommen werden. Obgleich die Dosierung und das Dosierungsschema in jedem Fall sorgsam abgewogen werden sollte, unter Anwendung gründlichen fachmännichen Urteils und unter Beachtung des Alters, die Gewichts und des Zustands des Patienten, der Art und der Schwere der Erkrankung, wird die Dosierung gewöhnlich in einem Bereich zwischen etwa 1 bis etwa $1 \times 10^4$ SIE/kg des Körpergewichts pro Tag liegen. In manchen Fällen wird man dabei eine ausreichende therapeutische Wirkung mit einer geringeren Dosis erreichen, während in anderen Fällen eine größere Dosis erforderlich sein wird.

Orale Applikation kann unter Verwendung fester und flüssiger Dosierungseinheiten durchgeführt werden, z. B. als Pulver, Tabletten, Dragees, Kapseln, Granulate, Suspensionen, Lösungen und dergleichen.

Pulver wird durch Zerkleinerung der Substanz in einer geeigneten Größe und Vermischen mit einem ebenfalls zerkleinerten pharmazeutischen Trägerstoff hergestellt. Obgleich ein eßbares Kohlenhydrat, wie z. B. Stärke, Lactose, Saccharose oder Glucose normalerweise zu diesem Zwecke Verwendung findet und auch hier benutzt werden kann, ist es wünschenswert ein nicht metabolisierendes Kohlenhydrat, wie z. B. ein Cellulosederivat zu benutzen.

Süßmittel, Geschmackszusätze, Konservierungsstoffe, Dispergiermittel und Färbemittel können auch mitverwendet werden.

Die Kapseln können durch Zubereitung der oben beschriebenen Pulvermischung und durch Füllung bereits gebildeter Gelatinehüllen hergestellt werden. Die Pulvermischung kann man vor dem Füllvorgang mit Gleitmitteln, wie z. B. Kieselgel, Talkum, Magnesiumstearat, Calciumstearat oder festem Polyäthylenglykol versetzen. Die Mischung kann man ebenfalls mit einem Desintegrator oder Lösungsvermittler, wie z. B. Agar-Agar, Calciumcarbonat oder Natriumcarbonat versetzen, um bei Einnahme der Kapsel die Zugänglichkeit des Inhibitors zu verbessern.

Die Anfertigung der Tabletten erfolgt z. B. durch Herstellung einer Pulvermischung, grob oder feinkörnig, und Hinzufügen eines Gleitmittels und Desintegrators. Aus dieser Mischung formt man Tabletten. Eine Pulvermischung bereitet man vor durch Mischung der Substanz, welche in geeigneter Weise zerkleinert wurde und ergänzt ein Verdünnungsmittel oder eine andere Trägersubstanz wie oben beschrieben. Gegebenenfalls fügt man ein Bindemittel hinzu: z. B. Carboxymethylcellulose, Alginate, Gelatine oder Polyvinylpyrrolidone, einen Lösungsverzögerer, wie z. B. Paraffin, einen Resorptionsbeschleuniger, wie z. B. ein quaternäres Salz, und/oder ein Adsorptionsmittel, wie z. B. Bentonit, Kaolin oder Dicalciumphosphat. Die Pulvermischung kann granuliert werden zusammen mit einem Bindemittel, wie z. B. Sirup, Stärkepaste, Akazienschleim, oder Lösungen aus Zellulose- oder Polymerenmaterialien. Danach preßt man das Produkt durch ein grobes Sieb. Als Alternative hierzu kann man die Pulvermischung durch eine Tablettenmaschine laufen lassen und die sich ergebenden ungleichmäßig geformten Stücke bis auf Korngröße zerkleinern. Damit die entstandenen Körner nicht in den tablettenbildenden Düsen stecken bleiben, kann man sie mit einem Gleitmittel versetzen, wie z. B. Stearinsäure, Stearatsalz, Talkum oder Mineralöl. Diese gleitfähig gemachte Mischung wird dann in Tablettenform gepreßt. Die Wirkstoffe können auch mit freifließenden inerten Trägerstoffen vereinigt werden und direkt in Tablettenform gebracht werden unter Auslassung der Granulat- oder Zerstückerungsschritte. Man kann das Produkt mit einer klaren oder opaken Schutzhülle versehen, z. B. einem Überzug aus Schellack, einem Überzug aus Zucker oder Polymersubstanzen und einer polierten Hülle aus Wachs. Farbstoffe können diesen Überzügen beigefügt werden, damit zwischen den verschiedenen Dosierungseinheiten unterschieden werden kann.

Die oral zu verabreichenden Zubereitungsformen, wie z. B. Lösungen, Sirup und Elixiere, lassen sich in Dosierungseinheiten herstellen, so daß eine bestimmte Menge Präparat eine bestimmte Menge Wirkstoff enthält. Sirup kann so hergestellt werden, daß der Wirkstoff in einer wäßrigen Lösung, welche geeignete Geschmacksstoffe enthält, gelöst wird; Elixiere werden unter Verwendung nichttoxischer, alkoholischer Trägerstoffe erhalten. Suspensionen kann man durch Dispergieren der Verbindung in einem nicht toxischen Trägerstoff darstellen. Lösungsvermittler und Emulgiermittel, wie z. B. äthoxylierte Isostearylalkohole und Polyoxyäthylensorbitester, Konservierungsmittel, geschmacksverbessernde Zusätze wie z. B. Pfefferminzöl oder Saccharin und dergleichen können auch zugegeben werden.

Dosierungsvorschriften können auf der Kapsel angegeben werden. Überdies kann die Dosierung so abgesichert sein, daß der Wirkstoff verzögert abgegeben wird, z. B. durch Einhalten des Wirkstoffes

in Polymerensubstanzen, Wachsen oder dergleichen.

Zusätzlich zu den oben erwähnten pharmazeutischen Zusammensetzungen lassen sich auch diese Wirkstoffe enthaltende Lebensmittel herstellen: beispielsweise Zucker, Brot, Kartoffelprodukte, Fruchtsaft, Bier, Schokolade und andere Konfektartikel, und Konserven, wie z. B. Marmelade, wobei zu diesen Produkten eine therapeutischwirksame Menge mindestens eines der erfindungsgemäßen Inhibitoren gegeben wurde.

Die unter Verwendung der erfindungsgemäßen Wirkstoffe hergestellten Nahrungsmittel eignen sich sowohl zur Diät bei Patienten, die an Stoffwechselstörungen leiden als auch zur Ernährung gesunder Personen im Sinne einer Stoffwechselstörungen vorbeugenden Ernährungsweise.

Die erfindungsgemäßen Verbindungen weisen weiterhin die Eigenschaft auf, in Tieren das Verhältnis des Anteiles an unerwünschtem Fett zum Anteil des erwünschten fettarmen Fleisches (mageres Fleisch) zugunsten des mageren Fleisches in hohem Maße zu beeinflussen. Dies ist von besonderer Bedeutung für die Aufzucht und Haltung von landwirtschaftlichen Nutztieren, z. B. in der Schweinemast, aber auch von erheblicher Bedeutung für die Aufzucht und Halterung von sonstigen Nutztieren und Ziertieren. Die Verwendung der Inhibitoren kann weiterhin zu einer erheblichen Rationalisierung der Fütterung der Tiere führen, sowohl zeitlich, mengenmäßig wie auch qualitätsmäßig. Da sie eine gewisse Verzögerung der Verdauung bewirken, wird die Verweildauer der Nährstoffe im Verdauungstrakt verlängert, wodurch eine mit weniger Aufwand verbundene ad libitum-Fütterung ermöglicht wird. Weiterhin ergibt sich bei der Verwendung der erfindungsgemäßen Inhibitoren in vielen Fällen eine erhebliche Einsparung von wertvollem Proteinfutter.

Die Wirkstoffe können somit praktisch in allen Bereichen der Tierernährung als Mittel zur Reduzierung des Fettansatzes sowie der Einsparung von Futtereiweiß verwendet werden.

Die Wirksamkeit der Wirkstoffe ist hierbei weitgehend unabhängig von der Art und dem Geschlecht der Tiere. Besonders wertvoll erweisen sich die Wirkstoffe bei Tierarten, die überhaupt oder in bestimmten Lebensabschnitten zu stärkerer Fetteinlagerung neigen.

Als Tiere, bei denen die Inhibitoren zur Reduzierung des Fettansatzes und/oder zu Einsparung von Futtereiweiß eingesetzt werden können, seien beispielsweise folgende Nutz- und Ziertiere genannt: Warmblüter wie Rinder, Schweine, Pferde, Schafe, Ziegen, Katzen, Hunde, Kaninchen, Pelztiere, z. B. Nerze, Chinchilla, andere Ziertiere, z. B. Meerschweinchen und Hamster, Labor- und Zootiere, z. B. Ratten, Mäuse, Affen usw. Geflügel, z. B. Broiler, Hühner, Gänse, Enten, Truthähne, Tauben, Papageien und Kanarienvögel und Kaltblüter, wie Fische, z. B. Karpfen und Reptilien, z. B. Schlangen.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,5 mg bis 2,5 g, insbesondere 10 bis 100 mg/kg Futter pro Tag. Die Dauer der Verabreichung kann von wenigen Stunden oder Tagen bis zu mehreren Jahren betragen. Die passende Menge Wirkstoff sowie die passende Dauer der Verabreichung stehen in engem Zusammenhang mit dem Fütterungsziel. Sie hängen insbesondere von der Art, dem Alter, dem Geschlecht, dem Gesundheitszustand und der Art der Haltung der Tiere ab und sind durch jedem Fachmann leicht zu ermitteln.

Die erfindungsgemäßen Wirkstoffe werden den Tieren nach den üblichen Methoden verabreicht. Die Art der Verabreichung hängt insbesondere von der Art, dem Verhalten und dem Allgemeinzustand der Tiere ab. So kann die Verabreichung einmal oder mehrmals täglich, in regelmäßigen oder unregelmäßigen Abständen, oral erfolgen. Aus Zweckmäßigkeitsgründen ist in den meisten Fällen eine orale Verabreichung, insbesondere im Rhytmus der Nahrungs- und/oder Getränkeaufnahme der Tiere, vorzuziehen.

Die Wirkstoffe können als reine Stoffe oder in formulierter Form verabreicht werden, wobei die formulierte Form sowohl als Premix, also in Mischung mit nichttoxischen inerten Trägerstoffen beliebiger Art, als auch als Teil einer Gesamtration in Form eines Beifutters bzw. als Mischungsbestandteil eines alleinigen Mischfutters zu verstehen ist. Mit eingeschlossen ist auch die Applikation geeigneter Zubereitungen über das Trinkwasser.

Die Wirkstoffe können gegebenenfalls in formulierter Form auch zusammen mit anderen Nähr- und Wirkstoffen, z. B. Mineralsalzen, Spurenelementen, Vitaminen, Eiweißstoffen, Energieträgern (z. B. Stärke, Zucker, Fette), Farbstoffen und/oder Geschmacksstoffen oder anderen Futterzusatzstoffen, wie z. B. Wachstumsförderern, in geeigneter Form verabreicht werden. Die Wirkstoffe können den Tieren vor, während oder nach der Nahrungsaufnahme gegeben werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwassers zugegeben werden.

Die Wirkstoffe können nach üblichen Methoden durch einfaches Mischen als reine Stoffe, vorzugsweise in fein verteilter Form oder in formulierter Form in Mischung mit eßbaren, nichttoxischen Trägerstoffen, gegebenenfalls auch in Form eines Premix oder eines Futterkonzentrates, dem Futter und/oder dem Trinkwasser beigefügt werden.

Das Futter und/oder Trinkwasser kann beispielsweise die erfindungsgemäßen Wirkstoffe in einer Konzentration von etwa 0,001 bis 5,0%, insbesondere 0,02 bis 2,0% (Gewicht) enthalten. Die optimale Höhe der Konzentration des Wirkstoffs im Futter und/oder Trinkwasser ist insbesondere abhängig von

der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Eiweißstoffen, einschließlich Vitaminen und Mineralstoffen enthalten. Das Futter kann sich beispielsweise zusammensetzen aus pflanzlichen Stoffen, z. B. Ölkuchenschroten, Getreideschroten, Getreidenebenprodukten, aber auch aus Heu, Gärfutter, Rüben und anderen Futterpflanzen, aus tierischen Stoffen, z. B. Fleisch- und Fischprodukten, Knochenmehl, Fetten, Vitaminen, z. B. A, D, E, K und B-Komplex sowie speziellen Proteinquellen, z. B. Hefen sowie bestimmten Aminosäuren und Mineralstoffen und Spurenelementen, wie z. B. Phosphor und Eisen, Zink, Mangan, Kupfer, Kobalt, Jod, usw.

Premixe können vorzugsweise etwa 0,1 bis 50%, insbesondere 0,5 bis 5,0% (Gewicht) an erfindungsgemäßen Verbindungen neben beliebigen eßbaren Trägerstoffen und/oder Mineralsalzen, z. B. kohlensaurem Futterkalk enthalten und werden nach den üblichen Mischmethoden hergestellt.

Mischfutter enthalten vorzugsweise 0,001 bis 5,0%, insbesondere 0,02 bis 2,0% (Gewicht) an erfindungsgemäßen Verbindungen neben den üblichen Rohstoffkomponenten eines Mischfutters, z. B. Getreideschroten oder-nebenprodukten, Ölkuchenschroten, tierischem Eiweiß, Mineralien, Spurenelementen und Vitaminen. Sie können nach den üblichen Mischmethoden hergestellt werden.

Vorzugsweise in Premixen und Mischfuttermitteln können die Wirkstoffe gegebenenfalls auch durch ihre Oberfläche bedeckende geeignete Mittel, z. B. mit nichttoxischen Wachsen oder Gelatine vor Luft, Licht und/oder Feuchtigkeit geschützt werden.

Beispiel für die Zusammensetzung eines fertigen Mischfutters für Geflügel, das einen erfindungsgemäßen Wirkstoff enthält:

200 g Weizen, 340 g Mais, 360,3 g Sojaschrot, 60 g Rindertalg, 15 g Dicalciumphosphat, 10 g Calciumcarbonat, 4 g jodiertes Kochsalz, 7,5 g Vitamin-Mineral-Mischung und 3,2 g Wirkstoff-Premix ergeben nach sorgfältigem Mischen 1 kg Futter.

Die Vitamin-Mineral-Mischung besteht aus:

6000 I. E. Vitamin A, 1000 I. E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg Mn $SO_4 \times H_2O$, 140 mg Zn $SO_4 \times 7\ H_2O$, 100 mg Fe $SO_4 \times 7\ H_2O$ und 20 mg Cu $SO_4 \times 5\ H_2O$. Der Wirkstoff-Premix enthält z. B. die Verbindung nach Beispiel 1 in der gewünschten Menge, z. B. 1600 mg und zusätzlich 1 g DL-Methionin sowie so viel Sojabohnenmehl, daß 3,2 g Premix entstehen.

Beispiel für die Zusammensetzung eines Schweinemischfutters, das einen Wirkstoff der Formel (I) enthält:

630 g Futtergetreideschrot (zusammengesetzt aus 200 g Mais-, 150 g Gerste-, 150 g Hafer- und 130 g Weizenschrot), 80 g Fischmehl, 60 g Sojaschrot, 58,8 g Tapiokamehl, 38 g Bierhefe, 50 g Vitamin-Mineral-Mischung für Schweine (Zusammensetzung, z. B. wie beim Kükenfutter) 30 g Leinkuchenmehl, 30 g Maiskleberfutter, 10 g Sojaöl, 10 g Zuckerrohrmelasse und 2 g Wirkstoff-Premix (Zusammensetzung z. B. beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Die Inhibitoren können einzeln oder auch in beliebigen Mischungen untereinander verwendet werden.

## Saccharase-Inhibitionstest in vitro

Der Saccharase-Inhibitionstest in virto ermöglicht die Bestimmung der enzyminhibitorischen Aktivität einer Substanz durch den Vergleich der Aktivität des solubilisierten intestinalen Disaccharidasen-Komplexes in Gegenwart bzw. in Abwesenheit (sog. 100%-Wert) des Inhibitors. Als Substrat, welches die Spezifität des Inhibitionstestes bestimmt, dient dabei eine praktisch Glucose-freie Saccharose (Glucose $<100$ ppm); die Enzymaktivitätsbestimmung basiert auf der spektrophotometrischen Bestimmung freigesetzter Glucose mittels Glucose-Dehydrogenase und Nicotinamid-adenindinucleotid als Cofaktor.

Eine Saccharase-Inhibitor-Einheit (SIE) ist definiert als diejenige inhibitorische Aktivität, welche in einem definierten Testansatz eine vorgegebene saccharolytische Aktivität um eine Einheit (Saccharase-Einheit = SE) reduziert; die Saccharase-Einheit ist dabei als diejenige Enzymaktivität definiert, welche unter vorgegebenen Bedingungen ein µmol Saccharose pro Minute spaltet und damit zur Freisetzung von je ein µmol Glucose, welche im Test bestimmt wird, und Fructose, welche im Test nicht erfaßt wird, führt.

Der intestinale Disaccharidasen-Komplex wird aus Schweinedünndarm-Mucosa durch tryptische Verdauung, Fällung aus 66% Äthanol bei $-20°C$, Aufnehmen des Präcipitates in 100 mM Phosphat-Puffer, pH 7,0 und abschließende Dialyse gegen denselben Puffer gewonnen.

10 µl einer Probelösung, die so angesetzt ist, daß die Extinktion des Testansatzes mindestens 10%, jedoch nicht mehr als 25% unter der des 100%-Wertes liegt, werden mit 100 µl einer Verdünnung des intestinalen Disaccharidasen-Komplexes in 0,1 M Maleinat-Puffer, pH 6,25, versetzt und für 10 Minuten bei 37°C vorinkubiert. Die Verdünnung des Disaccharidasen Komplexes ist auf eine Aktivität von 0,1 SE/ml einzustellen.

Anschließend wird die saccharolytische Reaktion durch Zugabe von 100 µl einer 0,4 M Lösung von Saccharose (»SERVA 35 779«) in 0,1 M Maleinat-Puffer, pH 6,25 gestartet und nach einer Inkubationsdauer von 20 Minuten bei 37°C durch die Zugabe von 1 ml Glucose-Dehydrogenase-Reagenz (1 Fläschchen Glucose-Dehydrogenase-Mutarotase-Gemisch lyophiliert (»MERCK 14 053«) und 331,7 mg β-Nicotinamid-adenin-dinucleotid (freie Säure, »BOEHRINGER« Reinheitsgrad I) in 250 ml 0,5 M Tris-Puffer, pH 7,6 gelöst) abgestoppt. Zum Nachweis der Glucose wird 30 Minuten bei 37°C inkubiert und schließlich bei 340 nm gegen einen Reagenzienblank (mit Enzym, jedoch ohne Saccharose) photometriert.

Die Berechnung der Hemmaktivität von Inhibitoren ist dadurch erschwert, daß schon geringfügige Änderungen im Testsystem, beispielsweise ein geringfügig von Bestimmung zu Bestimmung variierender 100%-Wert, von nicht mehr zu vernachlässigendem Einfluß auf das Testergebnis sind. Man umgeht diese Schwierigkeiten, indem man bei jeder Bestimmung einen Standard mitlaufen läßt; als Standard dient ein Saccharase-Inhibitor der Formel $C_{25}H_{43}O_{18}N$, welcher eine spezifische Hemmaktivität von 77 700 SIE/g aufweist und bei eingesetzten Mengen von 10 bis 20 mg im Test zu einer Hemmung von oben spezifizierter Größenordnung führt. Bei Kenntnis der Differenz der Extinktionen bei 340 nm von 100%-Wert und durch Standard gehemmtem Ansatz läßt sich aus der Extinktionsdifferenz von 100%-Wert und durch die Probelösung gehemmtem Ansatz unter Berücksichtigung der eingesetzten Menge an Inhibitor in bekannter Weise dessen spezifische Hemmaktivität errechnen, ausgedrückt in Saccharase-Inhibitor-Einheiten pro Gramm (SIE/g).

Die in den folgenden Beispielen angegebenen Rf-Werte der Dünnschichtchromatographie wurden auf DC-Fertigplatten Merck, Kieselgel 60 F 254, Eluiermittel Essigsäureäthylester/Methanol/Wasser/$NH_4OH$: 100/60/25/1, Detektion 1%ige wässrige $KMnO_4$-Lösung bestimmt.

## Beispiel 1

### 2-Hydroxymethyl-3,4,5-trihydroxy-6-methyl-piperidin

3,8 g 2-Hydroxymethyl-3,4,5-trihydroxy-6-cyan-piperidin (20 mMol) werden in 20 ml Hexamethyldisilazan suspendiert und mit 0,3 g Imidazol versetzt. Anschließend wird die Mischung 2,5 Stunden unter Rühren auf 60°C erwärmt. Die klare Lösung wird im Vakuum eingedampft, der Rückstand in 100 ml absolutem Diethylether aufgenommen und unter Rühren mit 50 mMol einer etherischen Methylmagnesiumjodidlösung (~75 ml) tropfenweise versetzt. Dabei bildet sich ein Niederschlag. Es werden weitere 2 Stunden bei Raumtemperatur gerührt. Der Ansatz wird mit 250 ml Eiswasser und 10 ml konz. Salzsäure versetzt und weitere 18 Stunden bei Raumtemperatur gerührt. Die wäßrige Phase wird anschließend abgetrennt, mit verdünnter Natronlauge neutralisiert und im Vakuum eingedampft. Der erhaltene Sirup wird in Methanol aufgenommen, das unlösliche Salze abgetrennt und das Filtrat wieder im Vakuum eingedampft. Diese Prozedur wird wiederholt und ergibt 6—10 g Sirup. Der Sirup wird in 50 ml Wasser gelöst und auf eine Dowex 50 WX 4 ($H^+$-Form) Austauscher-Säule (Ø = 2,5 cm, L = 30 cm) aufgetragen. Es wird zunächst mit 1,0 l Wasser gewaschen und dann mit 2% Ammoniak-Lösung eluiert. 2,8 g Rohprodukt (Gemisch der α- und β-Form) werden erhalten. Die Ionenaustauschertrennung wird wiederholt, wobei mit 0,1% Ammoniak eluiert wird. Man isoliert zuerst 0,1 g β-Form als nichtkristallines Harz, dann 2,3 g kristalline α-Form (d. s. 69% d. Th.), die nach Umkristallisation aus Methanol bei 172°C schmilzt;

Dünnschichtchromatographie: DC-Fertigplatten Merck, Kieselgel 60 F 254
Eluiermittel: Essigester/Methanol/Wasser/$NH_4OH$ = 100/60/25/1
Detektion: 1% wäßrige $KMnO_4$-Lösung
Rf = 0,25

In analoger Weise werden die folgenden Verbindungen mit Hilfe der entsprechenden metallorganischen Verbindungen erhalten.

## Beispiel 2

### 2-Hydroxymethyl-3,4,5-trihydroxy-6-äthyl-piperidin

Aus 3,8 g 2-Hydroxymethyl-3,4,5-trihydroxy-6-cyanpiperidin werden 0,1 g β-Form und 2,2 g (57% d. Th.) α-Form als farblose Kristalle vom Schmp. 160°C erhalten; $R_f$ = 0,47 (Bedingungen s. Beispiel 1).

Beispiel 3

2-Hydroxymethyl-3,4,5-trihydroxy-6-propyl-piperidin

Aus 3,8 g 2-Hydroxymethyl-3,4,5-trihydroxy-6-cyanpiperidin werden 0,6 g $\beta$-Form ($R_f = 0,6$) und 2,7 g (d. s. 65% der Theorie) $\alpha$-Form als farbloser Sirup erhalten.

MS = 174 m/e (M-$CH_2OH$)
$R_f = 0,40$ (Bedingungen s. Beispiel 1)

Beispiel 4

2-Hydroxymethyl-3,4,5-trihydroxy-6-n-butyl-piperidin

Aus 3,8 g 2-Hydroxymethyl-3,4,5-trihydroxy-6-cyanpiperidin werden 2,0 g (45% d. Th.) der kristallinen $\alpha$-Form erhalten. Nach Umkristallisation aus Cyclohexan, farblose Prismen vom Schmp. 143° C; $R_f = 0,55$ (Bedingungen s. Beispiel 1).

Beispiel 5

2-Hydroxymethyl-3,4,5-trihydroxy-6-n-pentyl-piperidin

Aus 3,8 g 2-Hydroxymethyl-3,4,5-trihydroxy-6-cyanpiperidin werden 0,8 g (17% d. Th.) der $\alpha$-Form als farbloser Sirup erhalten.

MS = 202 m/e (M—$CH_2OH$)
$R_f = 0,72$ (Bedingungen s. Beispiel 1)

Beispiel 6

2-Hydroxymethyl-3,4,5-trihydroxy-6-n-octyl-piperidin

Aus 3,8 g 2-Hydroxymethyl-3,4,5-trihydroxy-6-cyanpiperidin werden 2,3 g (41% d. Th.) kristalline $\alpha$-Form erhalten. Nach Umkristallisation aus Methanol farblose Nädelchen vom Schmp. 122° C; $R_f = 0,91$. (Bedingungen s. Beispiel 1)

Beispiel 7

2-Hydroxymethyl-3,4,5-trihydroxy-6-n-heptyl-piperidin

Aus 3,8 g 2-Hydroxymethyl-3,4,5-trihydroxy-6-cyan-piperidin werden 2,5 g (47,5% d. Th.) $\alpha$-Form erhalten. Nach Umkristallisation aus Ethanol kleine farblose Prismen vom Schmp. 144° C; $R_f = 0,82$ (Bedingungen s. Beispiel 1).

**Patentansprüche**

1. Verbindungen der allgemeinen Formel

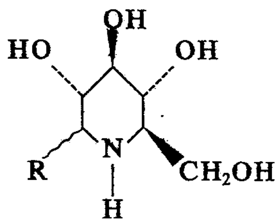

in der

R einen $C_1$—$C_{18}$-Alkyl-, $C_2$—$C_{18}$-Alkenyl- oder -Alkinylrest darstellt.

2. Verbindungen nach Anspruch 1 worin

R Methyl, Ethyl, Propyl, n-Butyl, n-Pentyl, n-Heptyl oder n-Octyl darstellt.

3. Verbindungen der Ansprüche 1 und 2 zur Behandlung von Adipositas, Diabetes und/oder Hyperlipämie.

4. Verfahren zur Herstellung von Verbindungen der Formel

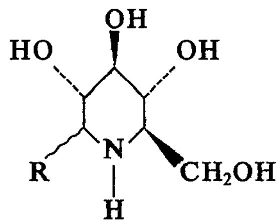

in der

R einen $C_1-C_{18}$-Alkyl, $C_2-C_{18}$-Alkenyl- oder -Alkinylrest darstellt,

dadurch gekennzeichnet, daß man 2-Hydroxymethyl-3,4,5-trihydroxy-5-cyano-piperidin durch Erwärmen mit Hexaalkyldisilazenen oder Trialkylsiliziumhalogeniden unter Zusatz eines Katalysators an den OH-Gruppen silyliert, mit einem geeigneten metallorganischen Reagenz der Formel

R—Y

worin

R die obengenannte Bedeutung hat und
Y ein Metall oder Halogenmetall bedeutet,

umsetzt und die Silyl-Schutzgruppen abspaltet.

5. Arzneimittel, gekennzeichnet durch einen Gehalt an einer Verbindung gemäß den Ansprüchen 1 oder 2 und gegebenenfalls pharmazeutisch geeigneten Zusatzstoffen.

6. Tierfuttermittel, gekennzeichnet durch einen Gehalt an einer Verbindung gemäß den Ansprüchen 1 oder 2.

**Claims**

1. Compounds of the general formula

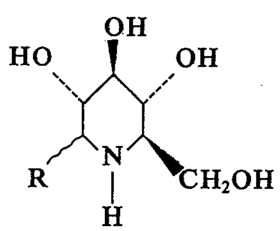

in which

R represents a $C_1-C_{18}$-alkyl, $C_2-C_{18}$-alkenyl or $C_2-C_{18}$-alkinyl radical.

2. Compounds according to Claim 1, wherein

R represents methyl, ethyl, propyl, n-butyl, n-pentyl, n-heptyl or n-octyl.

3. Compounds of Claims 1 and 2 for the treatment of adiposity, diabetes and/or hyperlipaemia.

4. Process for the preparation of compounds of the formula

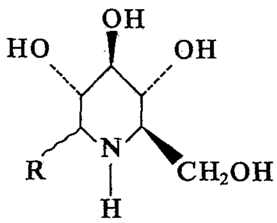

in which

R represents a $C_1-C_{18}$-alkyl, $C_2-C_{18}$-alkenyl or $C_2-C_{18}$-alkinyl radical,

characterised in that 2-hydroxymethyl-3,4,5-trihydroxy-6-cyano-piperidine is silylated on the OH groups by warming, with the addition of a catalyst, with hexaalkyldisilazenes or trialkylsilicon halides, the product is reacted with a suitable organometallic reagent of the formula

R—Y

wherein

R has the abovementioned meaning and
Y denotes a metal or halogeno-metal,

and the silyl protective groups are split off.

5. Medicaments, characterised in that they contain a compound according to Claims 1 or 2 and if appropriate pharmaceutically suitable additives.

6. Animal feedstuff, characterised in that it contains a compound according to Claims 1 or 2.

**Revendications**

1. Composés de formule générale:

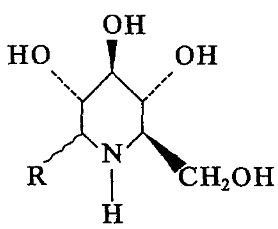

dans laquelle:

R est un reste alkyle en $C_1$ à $C_{18}$, alcényle ou alcynyle en $C_2$ à $C_{18}$.

2. Composés suivant la revendication 1, dans lesquels:

R est un reste méthyle, éthyle, propyle, n-butyle, n-pentyle, n-heptyle ou n-octyle.

3. Composés suivant les revendications 1 et 2, destinés au traitement de l'adiposité, du diabète et/ou de l'hyperlipidémie.

4. Procédé de production de composés de formule:

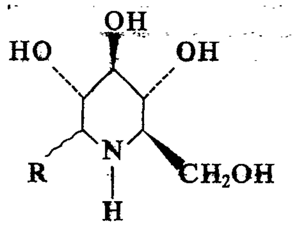

dans laquelle

R est un reste alkyle en $C_1$ à $C_{18}$, alcényle ou alcynyle en $C_2$ à $C_{18}$,

caractérisé en ce qu'on silyle sur les groupes OH la 2-hydroxyméthyl-3,4,5-trihydroxy-6-cyano-pipéridine par chauffage avec des hexaalkyldisilazènes ou des halogénures de trialkylsilicium avec addition d'un catalyseur, on l fait réagir avec un réactif organométallique approprié de formule:

R—Y

dans laquelle

R a la définition indiquée ci-dessus et
Y est un métal ou un métal halogéné,

et on élimine les groupes silyle protecteurs.

5. Médicament, caractérisé par une teneur en un composé suivant les revendications 1 ou 2 avec, le cas échéant, des additifs pharmaceutiquement appropriés.

6. Compositions pour l'alimentation du métal, caractérisées par une teneur en un composé suivant les revendications 1 ou 2.